# EUROPÄISCHE PATENTANMELDUNG

(11) **EP 3 241 615 A1**
(43) Veröffentlichungstag der Anmeldung: **08.11.2017**
(21) Anmeldenummer: 16168256.2
(22) Anmeldetag: 04.05.2016
(51) Int. Cl.: B05B 11/00, B65D 50/04, A61M 15/00

(54) **FLÜSSIGKEITSSPENDER**

(71) Anmelder: Aptar Radolfzell GmbH, 78315 Radolfzell (DE)
(72) Erfinder: Greiner-Perth, Jürgen, 78244 Gottmadingen (DE)
(74) Vertreter: Patentanwaltskanzlei Cartagena

(57) **Zusammenfassung**

Bekannt sind Spender (10) zum Austrag pharmazeutischer Flüssigkeiten mit einer Austragvorrichtung (20) mit einem Flüssigkeitsspeicher (22) und einer Austragöffnung (24) zum Austrag der Flüssigkeit sowie mit einer Kappe (50), die auf der Austragvorrichtung (20) in einer Aufsetz-richtung (2a) aufsetzbar ist und im aufgesetzten Zustand die Austragöffnung (24) überdeckt.

Hierzu wird vorgeschlagen, dass die Kappe (50) über eine stirnseitige Deckfläche (52) und eine damit verbundene Mantelfläche (54) verfügt. An der Mantelfläche (54) ist mindestens ein Wippelement (70) vorgesehen. Das Wippelement (70) ist stoffschlüssig und um eine bezogen auf die Aufsetzrichtung (2a) tangentiale Kippachse (4) schwenkbeweglich an der Mantelfläche (54) angebracht, wobei die Kippachse (4) das Wippelement (70) in einen Betätigungsschenkel (72) und einen Rastschenkel (76) unterteilt, so dass das Niederdrücken des Betätigungsschenkels (72) eine gegengerichtete Verlagerung des Rastschenkels (76) bewirkt. An der Innenseite des Rastschenkels (76) ist eine Halteraste (78) vorgesehen, die gemeinsam mit einer korrespondierenden Haltekante (30) an der Austragvorrichtung (20) formschlüssig ein Abziehen der Kappe (50) entgegen der Aufsetzrichtung (2a) verhindert.

## Beschreibung

Die Erfindung betrifft einen Spender zum Austrag pharmazeutischer Flüssigkeiten. Ein solcher gattungsgemäßer Spender umfasst eine Austragvorrichtung mit einem Flüssigkeitsspeicher und einer Austragöffnung zum Austrag der Flüssigkeit sowie eine Kappe, die auf der Austragvorrichtung in einer Aufsetzrichtung aufsetzbar ist und im aufgesetzten Zustand die Austragöffnung überdeckt.

Gattungsgemäße Spender werden verwendet, um hiermit pharmazeutische Flüssigkeiten auszugeben. Hierbei kann es sich um hochviskose cremeartige Flüssigkeiten handeln, um Flüssigkeiten, die in Tropfenform ausgegeben werden, oder auch um Flüssigkeiten, die in Form eines Sprühstrahls abgegeben werden. Viele pharmazeutische Flüssigkeiten stellen für Kinder, insbesondere für kleine Kinder, eine erhebliche Gefahr dar, so dass es bekannt ist, die Kappen gattungsgemäßer Spender mit Merkmalen auszugestalten, die es Kindern schwer machen, die Kappen abzunehmen, um an die Flüssigkeit zu gelangen.

Spender mit solchen kindergesicherten Kappen sind jedoch häufig vergleichsweise aufwendig in der Herstellung und daher teuer. Weiterhin eignen sich viele bekannte Mechanismen zur Kindersicherung einer Kappe nicht gut für sogenannte Side-Actuation-Austragvorrichtungen. Hierbei handelt es sich um Austragvorrichtungen, die über einen Betätigungsdrücker verfügt, dessen Bewegungsrichtung nicht mit der Austragrichtung übereinstimmt, sondern insbesondere in etwa im rechten Winkel zur Austragrichtung eingedrückt wird.

### AUFGABE UND LÖSUNG

Aufgabe der Erfindung ist es, eine baulich besonders einfache Möglichkeit zu schaffen, einen gattungsgemäßen Spender gegen den Zugriff durch Kinder, insbesondere kleine Kinder, zu sichern.

Erfindungsgemäß ist vorgesehen, dass die Kappe eines gattungsgemäßen Spenders wie folgt ausgestaltet und auf die Austragvorrichtung angepasst ist. Die Kappe verfügt über eine stirnseitige Deckfläche und eine damit verbundene Mantelfläche. An dieser Mantelfläche ist mindestens ein Wippelement vorgesehen, das stoffschlüssig und um eine Kippachse schwenkbeweglich an der Mantelfläche angebracht ist.

Die Kippachse unterteilt das Wippelement in einen Betätigungsschenkel und einen Rastschenkel, so dass das Niederdrücken des Betätigungsschenkels eine gegengerichtete Verlagerung des Rastschenkels bewirkt. Bezogen auf eine Mittelachse der Kappe führt also ein radiales Eindrücken des Betätigungsschenkels hin zur Mittelachse zu einer radialen Beabstandung des Rastschenkels von der Mittelachse. Hierdurch wird die Kappe von der Austragvorrichtung gelöst und ist anschließend abziehbar. Um dies zu erreichen ist an der Innenseite des Rastschenkels eine Halteraste vorgesehen, die unausgelenkt gemeinsam mit einer korrespondierenden nach außen weisenden Haltekante an der Austragvorrichtungformschlüssig ein Abziehen der Kappe entgegen der Aufsetzrichtung verhindert.

Die genannte Mantelfläche umgibt im aufgesetzten Zustand einen Applikator des Spenders zumindest zum überwiegenden Teil. An dieser Mantelfläche ist einstückig das Wippelement vorgesehen, welches über einen Verbindungssteg oder nachfolgend noch beschriebene Torsionsbrücken mit der Mantelfläche derart verbunden ist, dass die beschriebene Kippachse definiert wird, um die das Wippelement elastisch verschwenkbar ist. Die Mantelfläche und das Wippelement bestehen vorzugsweise aus identischem Kunststoff. Denkbar sind jedoch auch Gestaltungen, die im Zweikomponenten-Verfahren gefertigt wurden, beispielsweise um ein starreres Wippelement mit einem elastischeren Verbindungsabschnitt mit der Mantelfläche einstückig verbunden ist.

Die Kippachse ist vorzugsweise bezogen auf die Aufsetzrichtung tangential ausgerichtet, also horizontal bei aufrechter Kappe mit nach unten weisender Aufsetzrichtung. Alternativ ist auch eine Gestaltung mit zur Aufsetzrichtung paralleler oder hiergegen um weniger als 15° angewinkelter Kippachse, denkbar. Auch sind allgemein Ausrichtungen der Kippachse in jener durch die Parallele und die Tangente aufgespannten Ebene von Vorteil, wenngleich in besonderen Fällen auch eine nicht in dieser Ebene liegende Ausrichtung der Kippachse vorteilhaft sein könnte.

Durch Druck auf den Betätigungsschenkel des Wippelements, insbesondere radial in Richtung auf den innen liegenden Applikator des Spenders zu, wird der Rastschenkel des Wippelements in entgegengesetzte Richtung, insbesondere nach außen, ausgelenkt. Hierdurch gelangt die am Rastschenkel vorgesehene Halteraste außer Eingriff von der an der Austragvorrichtung vorgesehenen Haltekante, so dass das Abziehen der Kappe anschließend möglich ist. Beim Aufsetzen der Kappe sorgt die elastische Verbindung zwischen Wippelement und Mantelfläche dafür, dass das Wippelement automatisch ausgelenkt wird, bevor die Halteraste unter der Haltekante einschnappt.

Grundsätzlich ist es möglich, die Kappe nur mit einem Wippelement zu versehen. Jedoch sind Gestaltungen mit mehreren Wippelementen, insbesondere zwei oder drei gleichmäßig oder ungleichmäßig über den Umfang verteilten Wippelementen im Sinne des Erschwerens des gleichzeitigen Eindrückens der jeweiligen Betätigungsabschnitte von Vorteil. Ungeachtet der Anzahl der Wippelemente wird es als vorteilhaft angesehen, wenn ein jedes Wippelement an seinem der Kippachse abgewandten Ende des Betätigungsschenkels mit mindestens 20 N beaufschlagt werden muss, um die jeweilige Halteraste und die Haltekante außer Eingriff voneinander zu bringen. Um die Handhabung zu erleichtern und für Erwachsene intuitiver zu machen, kann der Betätigungsschenkel mit einer außen liegenden Strukturierung, beispielsweise einer Riffelung, versehen sein.

Die auf Seiten der Austragvorrichtung vorgesehene Haltekante kann unterbrochen sein, so dass sie lediglich im Bereich der mindestens einen wippelementseitigen Halteraste vorgesehen ist. Von Vorteil ist jedoch eine umlaufende Haltekante, die orientierungsunabhängig das Aufsetzen und Verschnappen der Kappe mit der Haltekante gestattet.

Die Kappe überdeckt im aufgesetzten Zustand zumindest die Austragöffnung. Diese Austragöffnung kann beispielsweise am distalen Ende einer zur nasalen Anwendung vorgesehenen Applikatorspitze, einer sogenannten Nasenolive, vorgesehen sein.

Die Kappe kann als geschlossene Kappe ausgebildet sein, die auch ohne Berücksichtigung der Wippelemente im aufgesetzten Zustand einen Kappeninnenraum hermetisch gegenüber einer Umgebung abdichtet. Bei einer solchen Gestaltung sind die Wippelemente vorzugsweise außenseitig auf die geschlossene Mantelfläche aufgesetzt.

Im Sinne einer besonders kostengünstigen Herstellung kann jedoch eine andere Ausgestaltung vorgesehen sein, bei der in der Mantelfläche der Kappe eine Aussparung vorgesehen ist. In dieser Aussparung ist das Wippelement angeordnet und hierbei mittels eines Freischnittes von der Mantelfläche getrennt, wobei der Freischnitt einen Kappeninnenraum mit einer äußeren Umgebung kommunizierend verbindet. Das Wippelement kann im Bereich zweier Torsionsbrücken mit der Mantelfläche verbunden sein, wobei diese beiden Torsionsbrücken die Kippachse des Wippelements definieren, um die das Wippelement elastisch verkippbar ist.

Eine solche Kappe mit Wippelementen, die in Aussparungen der Mantelfläche vorgesehen sind, führen zu einer besonders preisgünstigen Herstellung. Zudem kann die Kappe bei einer solchen Ausgestaltung wenig voluminös ausgebildet sein und gestattet somit die Verwendung an kompakten Spendern. Die Mantelfläche einer solchen Kappe weist vorzugsweise eine im Wesentlichen rotationssymmetrische Form auf, die im Bereich der Aussparungen durch bündig hiermit fluchtende Wippelemente ergänzt ist. Diese bündig fluchtende Anordnung der Wippelemente erschwert es einem Kind, diese unmittelbar im Bereich des Rastschenkels zu ergreifen und nach außen zu ziehen.

Durch den Freischnitt, mit dem ein jedes Wippelement gegenüber der Mantelfläche getrennt ist und der vorzugsweise eine mittlere Breite <3mm, insbesondere <2mm, aufweist, ist ein Kappeninnenraum bzw. im Falle mehrerer Wippelemente ein gemeinsamer Kappeninnenraum mit einer äußeren Umgebung verbunden, so dass Luft in die Kappe einströmen kann. Dies wird bei einigen Spendern in Abhängigkeit des Anwendungszwecks als wünschenswert angesehen, damit der im Kappeninnenraum befindliche Applikator, ein Teil dessen und/oder die Austragöffnung auch bei aufgesetzter Kappe durch die Verbindung mit der Umgebung trocknen können. Es ist nicht erforderlich, dass der gesamte Kappeninnenraum durch den Freischnitt mit einer äußeren Umgebung kommunizierend verbunden ist, wie im Weiteren noch erläutert wird.

Das Wippelement kann in der Aussparung allseitig von der Mantelfläche eingerahmt sein. Bei einer solchen Gestaltung ist eine allseitig von Mantelfläche umgebende Aussparung vorgesehen, in der das Wippelement kippbeweglich vorgesehen ist. Dieses vollständige Umgeben des Wippelements durch die Mantelfläche erschwert es einem Kind, unmittelbar am Rastschenkel des Wippelements zu ergreifen und diesen nach außen zu ziehen, um die Kappe lösen zu können.

Die Aussparung kann sich stattdessen bis zu einem unteren Rand der Mantelfläche erstrecken, so dass das Wippelement in der Aussparung im Bereich der Aussparung das untere Ende der Kappe bildet. In diesem Zusammenhang ist mit unten jene Raumrichtung gemeint, in die die Aufsetzrichtung weist. Die Aussparung ist bei dieser Ausgestaltung nicht vollständig von der Mantelfläche umgeben, sondern nach unten offen. Dies erleichtert die Herstellung, kann es jedoch für ein Kind leichter machen, den Rastschenkel ohne Begreifen der Funktion des Betätigungsschenkels nach außen zu ziehen, um die Halteraste von der Haltekante zu lösen.

Dem kann mit einer Weiterbildung entgegengewirkt werden, bei der die Mantelfläche sich beidseitig der Aussparung weiter nach unten als das Wippelement erstrecken. Durch diese Erstreckung der beidseitigen benachbarten Mantelflächenabschnitte über das Wippelement hinaus nach unten wird es für ein Kind erschwert, den Rastschenkel nach außen zu ziehen.

Das Wippelement und die Mantelfläche können im Lieferzustand zusätzlich zur Verbindung im Bereich der Kippachse auch mittels einer Sicherungsbrücke miteinander verbunden sein. Diese muss zum Zwecke des Abnehmens der Kappe bestimmungsgemäß zerstört werden.

Diese zusätzliche Sicherungsbrücke führt aufgrund ihrer Beabstandung zur Kippachse dazu, dass das Wippelement im Lieferzustand zunächst nicht ausreichend beweglich ist, um die Kappe von der Austragvorrichtung abzuziehen. Die Sicherungsbrücke muss zunächst zerstört werden und stellt aufgrund dessen einen Originalitätsschutz dar, der Auskunft darüber gibt, ob der Spender bereits in Betrieb genommen wurde. Weiterhin stellt die Sicherungsbrücke im Lieferzustand eines Spenders eine wirksame zusätzliche Kindersicherung dar.

Die Kappe kann eine Stufe aufweisen, die bezogen auf die Aufsetzrichtung durch einen Sprung im Außendurchmesser von einem kleineren Querschnitt zu einem größeren Querschnitt gebildet wird. Beim Vorhandensein einer solchen Stufe ist es von Vorteil, wenn die Kippachse und insbesondere die zwei Torsionsbrücken fluchtend mit dem kleineren Querschnitt angeordnet sind.

Im Bereich der Stufe weitet sich die Kappe in Aufsetzrichtung vergleichsweise deutlich auf, worunter verstanden wird, dass bezogen auf einen Längsschnitt durch die Kappe deren Mantelfläche im Bereich der Stufe mindestens einen 45°-Winkel mit der Aufsetzrichtung einschließt. Eine solche Stufe erleichtert im Zuge der Montage die Vereinzelung von Kappen und deren bestimmungsgemäße Aufnahme in einer Vorrichtung zum Aufsetzen der Kappe. Der Bereich der Stufe eignet sich auch gut für die Anbringung der Kippachse, indem hier insbesondere die genannten Torsionsbrücken angeordnet sein können. Damit das Wippelement eine ausreichende Bewegungsfreiheit aufweist, hat es sich als vorteilhaft herausgestellt, die Torsionsbrücken am kleineren Querschnitt bzw. Durchmesser vorzusehen.

Wie bereits erläutert, können die Freischnitte, die eine kommunizierende Verbindung zwischen dem Kappeninnenraum und einer äußeren Umgebung schaffen, gewünscht sein, um ein Abtrocknen der Oberflächen der Austragvorrichtung auch bei aufgesetzter Kappe zu ermöglichen. Es sind jedoch auch Gestaltungen denkbar, bei denen zumindest ein Teil der Oberfläche der Austragvorrichtung, insbesondere die Austragöffnung und ein die Austragöffnung umgebender Bereich, gegenüber der Umgebung isoliert sein sollen. Um dies bei einer Kappe mit den genannten Freischnitten zu erzielen, kann Folgendes vorgesehen sein. Die Innenseite der Kappe und die Außenform derAustragvorrichtung können derart aufeinander angepasst sein, dass bei aufgesetzter Kappe ein Anliegen der Innenseite der Kappe an der Austragvorrichtung entlang eines umlaufend geschlossenen Kontaktbereichs bewirkt wird, durch den die Austragöffnung gegenüber einem mit der äußeren Umgebung kommunizierenden Bereich des Kappeninnenraums isoliert ist.

Bei einer solchen Gestaltung ist somit zumindest ein Teil des Kappeninnenraums gegenüber der äußeren Umgebung isoliert.

Der umlaufende Kontaktbereich kann auf Kappenseite durch eine kappenseitige erste Kontaktfläche und auf Seite der Austragvorrichtung durch eine austragsvorrichtungsseitige zweite Kontaktfläche gebildet sein. Hierbei können die Kontaktflächen mit der Aufsetzrichtung einen Winkel von <20° einschließen.

Durch den spitzen Winkel gestatten es die beidseitigen Kontaktflächen bei der Verwendung ausreichend weichen Kunststoffes, eine hermetische Abdichtung mit einer vergleichsweise großen Lagetoleranz herzustellen. Dies ist von Vorteil, da anderenfalls eine vermeidenswerte Doppelpasssung entstünde, da in Aufsetzrichtung auch die Halteraste und die Haltekante eine definierte Solllage der Kappe zur Austragvorrichtung vorgeben.

Die erste Kontaktfläche kann durch die Innenseite eines umlaufenden Ringsteges gebildet werden, der im Bereich der stirnseitigen Deckfläche innenseitig an der Kappe angebracht ist.

Die sich nach innen über die Stirnfläche der Kappe erhebende Struktur in Form eines Ringsteges erlaubt es, die Abdichtung, insbesondere im Bereich einer Nasenolive, zu erzielen, ohne dass die von außen sichtbare Kappenform einer entsprechenden Anpassung bedarf.

Die Austragvorrichtung umfasst üblicherweise eine Gehäuse sowie eine Steuereinrichtung mit einer Pumpe oder einem Schaltventil, das durch eine erste Leitung mit dem Flüssigkeitsspeicher und durch eine zweite Leitung mit der Austragöffnung verbunden ist und mittels derer Flüssigkeit aus dem Flüssigkeitsspeicher zur Austragöffnung geleitet werden kann. Sie umfasst weiterhin einen Betätigungsdrücker, der zum Zwecke des Flüssigkeitsaustrags kraftbeaufschlagbar ist und hierdurch die Steuereinrichtung betätigt.

Bei einer ersten besonderen Ausgestaltung ist die Austragöffnung ist an einem Applikator vorgesehen, der gemeinsam mit dem Betätigungsdrücker verlagerbar ist, wobei an diesem Applikator auch die Haltekante zur Festlegung der Kappe vorgesehen ist.

Bei dieser ersten Ausgestaltung wird die Kappe somit an einer Teileinheit der Austragvorrichtung angebracht, die den Applikator und den Betätigungsdrücker in sich vereinigt, welche gemeinsam gegenüber einem anderen Teil der Austragvorrichtung, insbesondere umfassend den Flüssigkeitsspeicher, verlagerbar ist. Der Spender bleibt somit auch bei aufgesetzter Kappe betätigbar, wobei hier insbesondere eine der oben und im weiteren beschriebenen abdichtenden Kappen Verwendung findet, damit dennoch keine Flüssigkeit ausgetragen werden kann.

Alternativ hierzu könnte bei einer derartigen Ausgestaltung der Austragvorrichtung mit einem gemeinsam mit dem Applikator und der Austragöffnung verlagerbaren Betätigungsdrücker die Kappe auch an einem nicht verlagerbaren Gehäuseabschnitt angebracht sein.

Bei einer anderen besonderen Gestaltung ist die Austragöffnung zum Austrag der Flüssigkeit in einer Austragrichtung ausgebildet, die in etwa orthogonal zu einer Radialrichtung ausgebildet ist, in der der Betätigungsdrücker zum Zwecke des Austrags eingedrückt werden kann. Die Kappe überdeckt dabei im aufgesetzten Zustand auch diesen Betätigungsdrücker.

Dieser Typ Austragrichtung wird üblicherweise mit Side-Actuation bezeichnet. Die erfindungsgemäße Ausgestaltung der Kappe eignet sich insbesondere für solche Austragvorrichtungen, weil viele andere Konstruktionen kindergesicherter Kappen aufgrund der asymmetrischen Ausgestaltung der Austragvorrichtung mit seitlich angebrachtem Betätigungsdrücker hier nicht gut verwendbar sind. Durch die Überdeckung auch des Betätigungsdrückers mittels der Kappe wird bei dieser Gestaltung von Spendern bei aufgesetzter Kappe bereits das Betätigen verhindert. Die Wippelemente der Kappe sind vorzugsweise derart an die Ausgestaltung und die Positionierung des Betätigungsdrückers angepasst, dass alleine durch Eindrücken des Betätigungsschenkels ein Eindrücken des Betätigungsdrückers nicht möglich ist.

In Hinblick auf die Anzahl der Wippelemente sind verschiedene Konfigurationen denkbar.

Bei einer ersten Variante weist die Kappe nur ein Wippelement auf. Die erforderliche Kraftbeaufschlagung des Betätigungsschenkels am kippachsenfernsten Punkt zur ausreichenden Auslenkung der Halteraste, um diese außer Eingriff von der Haltekante zu bringen, beträgt dabei vorzugsweise mindestens 30 N. Zwar wird die Gestaltung mit nur einem Wippelement nicht als ideal angesehen, da dieses bei gleichzeitigem Festhalten der Austragvorrichtung auch durch eine Kinderhand erreichbar ist. Durch eine ausreichend hohe erforderliche Kraftbeaufschlagung zum Zwecke der ausreichenden Auslenkung der Halteraste kann dies jedoch kompensiert werden.

Bei einer zweiten Variante weist die Kappe zwei Wippelemente auf. Die erforderliche Kraftbeaufschlagung der jeweiligen Betätigungsschenkel beträgt dann vorzugsweise mindestens 20 N. Die beiden Wippelemente können bei einer solchen Ausgestaltung vorzugsweise einander gegenüberliegend an der Kappe vorgesehen sein, so dass ein Erwachsener keine Schwierigkeit hat, beide Wippelemente bzw. die jeweiligen Betätigungsschenkel mit nur einer Hand, insbesondere mit Daumen und Zeigefinger dieser Hand, gleichzeitig einzudrücken. Die Betätigungsschenkel können durch eine einander gegenüberliegende Anordnungjedoch ausreichend weit voneinander entfernt sein, dass es mit der kleinen Hand eines Kindes nicht gelingt, diese gleichzeitig einzudrücken. Durch eine Betätigungskraft von mindestens 20 N wird es zusätzlich erschwert.

Bei einer dritten Variante weist die Kappe drei oder mehr Wippelemente auf. Durch die Verwendung von drei Wippelementen oder gar mehr wird es für ein Kind weiter erschwert, diese gleichzeitig zu betätigen. Für einen Erwachsenen kann dies jedoch auch mit nur einer Hand möglich sein, beispielsweise durch Verwendung von Daumen, Zeigefinger und Mittelfinger.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 bis 4 zeigen eine erste Variante eines erfindungsgemäßen Spenders, dessen Austragvorrichtung als Side-Actuation-Austragvorrichtung ausgebildet ist und der über eine Kappe mit freigeschnittenen Wippelementen verfügt.
Fig. 5 bis 7 zeigen eine zweite Variante eines erfindungsgemäßen Spenders, der mit einer belüftenden Kappe mit freigeschnittenen Wippelementen versehen ist.
Fig. 8 bis 11 zeigen eine dritte Variante eines erfindungsgemäßen Spenders, der mit einer belüftungsverhindernden Kappe mit freigeschnittenen Wippelementen versehen ist.
Fig. 12 bis 15 zeigen eine weitere Variante eines erfindungsgemäßen Spenders, der ebenfalls mit einer belüftungsverhindernden Kappe mit freigeschnittenen Wippelementen versehen ist.
Fig. 16 bis 19 zeigen eine fünfte Variante eines erfindungsgemäßen Spenders, der mit einer Kappe versehen ist, deren freigeschnittene Wippelemente durch die Länge des Mantels besonders schwer zu lösen sind.
Fig. 20 bis 23 zeigen eine sechste Variante eines erfindungsgemäßen Spenders, der mit einer Kappe versehen ist, deren freigeschnittene Wippelemente durch Anordnung in allseitig umgebenen Aussparungen besonders schwer zu lösen sind.
Fig. 24 zeigt eine siebte Variante eines erfindungsgemäßen Spenders, bei der die Wippelemente außenseitig auf dem Mantel angebracht sind.

### DETAILLIERTE BESCHREIBUNG DERAUSFÜHRUNGSBEISPIELE

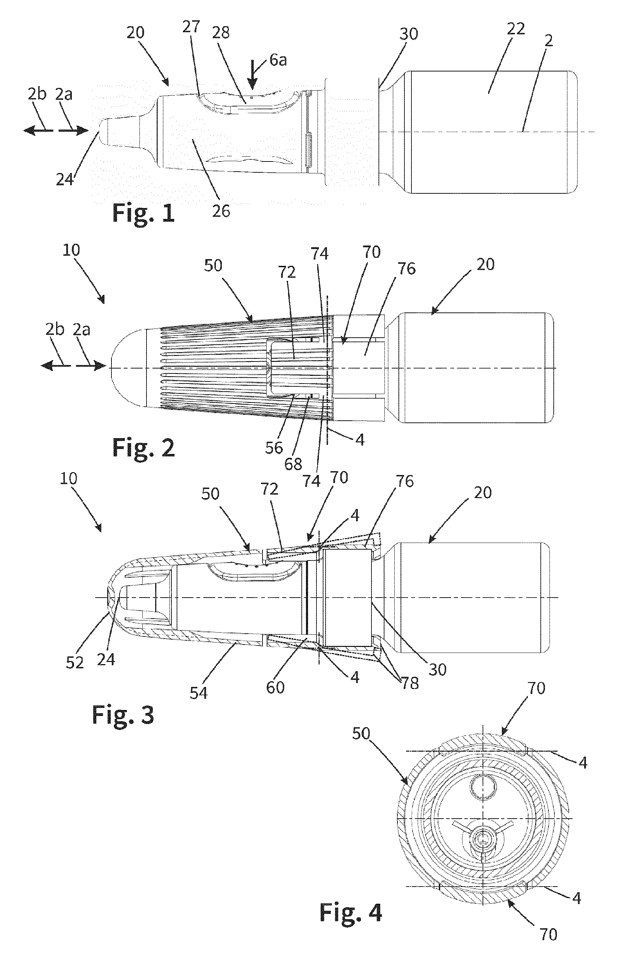

Die Fig. 1 bis 4 zeigen eine erste Ausgestaltung eines erfindungsgemäßen Spenders. Dieser Spender 10 verfügt über eine Austragvorrichtung 20, die separat in Fig. 1 dargestellt ist. Diese Austragvorrichtung 20 weist ein Gehäuse 26 mit einem Flüssigkeitsspeicher 22 auf. Am distalen Ende der Austragvorrichtung 20 ist eine Austragöffnung 24 vorgesehen, durch die pharmazeutische Flüssigkeit aus dem Flüssigkeitsspeicher 22 ausgetragen werden kann. Der Austrag kann beispielsweise in Tropfenform oder als Sprühstrahl erfolgen. In einer Mantelfläche des Gehäuses 26 ist eine Aussparung 27 vorgesehen, in der ein Betätigungsdrücker 28 angeordnet ist. Dieser kann zum Zwecke des Austrags in Richtung 6a eingedrückt werden. Er betätigt hierdurch eine nicht dargestellte Pumpeinrichtung, die Flüssigkeit zur Austragöffnung 24 pumpt. Stattdessen ist auch eine Gestaltung denkbar, bei der die Flüssigkeit im Flüssigkeitsspeicher 22 unter Druck gehalten wird und mittels des Betätigungsdrückers 28 ein Auslassventil geöffnet wird. Diese Form einer Austragvorrichtung nennt man auch Side-Actuation-Austragvorrichtung, da der Betätigungsdrücker 28 in einer zur Austragrichtung 2b in einer etwa orthogonalen Richtung eingedrückt wird.

Der Spender 10 umfasst weiterhin eine Kappe 50, die in den Fig. 2 bis 4 dargestellt ist. Diese Kappe 50 ist bestimmungsgemäß als kindergesicherte Kappe ausgestaltet. Sie soll demnach verhindern, dass insbesondere kleine Kinder in der Lage sind, Flüssigkeit mittels der Austragvorrichtung 20 auszutragen. Die Kappe 50 überspannt zu diesem Zweck sowohl die Austragöffnung 24 als auch den Betätigungsdrücker 28. Sie ist zudem durch Halterasten 78 gegen Abziehen gesichert. Diese Halterasten 78 sind am distalen Ende von Wippelementen 70 vorgesehen, welche mittels zweier Brücken 74 mit einer Mantelfläche 54 der Kappe 50 einstückig verbunden sind. Die Torsionsbrücken 74 definieren eine Kippachse 4, um die das Wippelement 70 als Ganzes schwenkbeweglich ist, wenn es im Bereich eines Betätigungsschenkels 72 in Richtung der Mittelachse 2 des Spenders niedergedrückt wird. Ein solches Niederdrücken bewirkt, dass die gegenüberliegenden Rastschenkel 76 radial nach außen abgehoben werden und hierdurch außer Eingriff von einer Haltekante 30 der Austragvorrichtung 20 gelangen. Um diese Kippbewegung zu ermöglichen, ist ein Kappeninnenraum 60 der Kappe ausreichend groß gestaltet.

Durch die Gestaltung der Kappe 50 mit zwei Wippelementen 70 ist es einem Kind kaum möglich, mit nur einer Hand die beiden Betätigungsschenkel 72 mit der erforderlichen Kraft von jeweils 20 N einzudrücken. Verwendet das Kind dagegen zwei Hände, so fällt es ihm schwer, die Kappe 50 von der Austragvorrichtung 20 abzuziehen. Es ist somit auf einfache Weise eine kindergesicherte Gestaltung geschaffen, die insbesondere auch Anforderungen an eine geringe Kappengröße erfüllt.

Bei der Ausgestaltung gemäß der Fig. 1 bis 4 ist bewusst vorgesehen, dass die Umgebung der Austragöffnung 24 kommunizierend mit allen anderen Teilen des Kappeninnenraums 60 verbleibt, so dass durch die Freischnitte 68, welche abseits der Torsionsbrücken 74 die Wippelemente 70 und die Mantelfläche 54 voneinander trennen, Luft in die Kappe einströmen und bis zur Austragöffnung 24 gelangen kann. Auf diese Weise trocknen die Austragöffnung und deren Umgebung nach Verwendung des Spenders auch bei aufgesetzter Kappe ab, wodurch Bakterienwachstum hier vermieden wird.

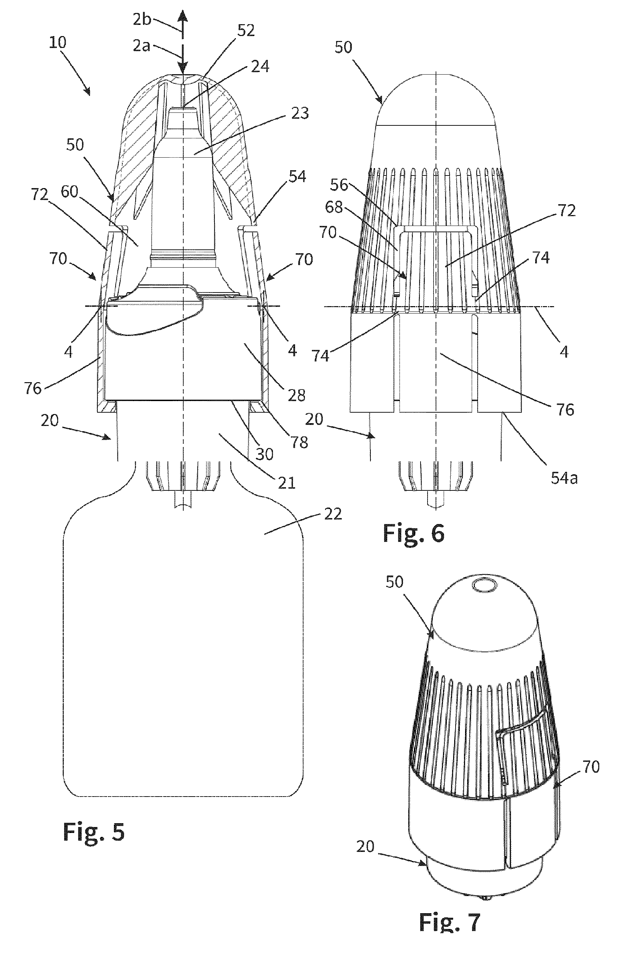

Die Kappe gemäß der Fig. 5 bis 7 entspricht baulich weitgehend der Kappe 50 des ersten Ausführungsbeispiels. Abweichend hiervon wird die Kappe 50 hier bei einer Austragvorrichtung 20 verwendet, die über eine gemeinsame Baueinheit verfügt, welche den Betätigungsdrücker 28, die einen Applikator 23 in Form einer Nasenolive und die Austragöffnung 24 umfasst. Auch bei einer solchen Gestaltung einer Austragvorrichtung ist die Verwendung der Kappe sinnvoll. Die Halterasten 78 an den Schenkeln 76 der Wippelemente 70 sind im Falle dieser Austragvorrichtung an einer Haltekante 30 festgelegt, die den Betätigungsdrücker 28 am unteren Ende abschließt. Die genannte Baueinheit umfassend den Betätigungsdrücker 28 kann gegenüber einer Basis 21 zum Zwecke des Austrags niedergedrückt werden. Dies ist beim dargestellten Spender daher auch bei aufgesetzter Kappe 50 möglich.

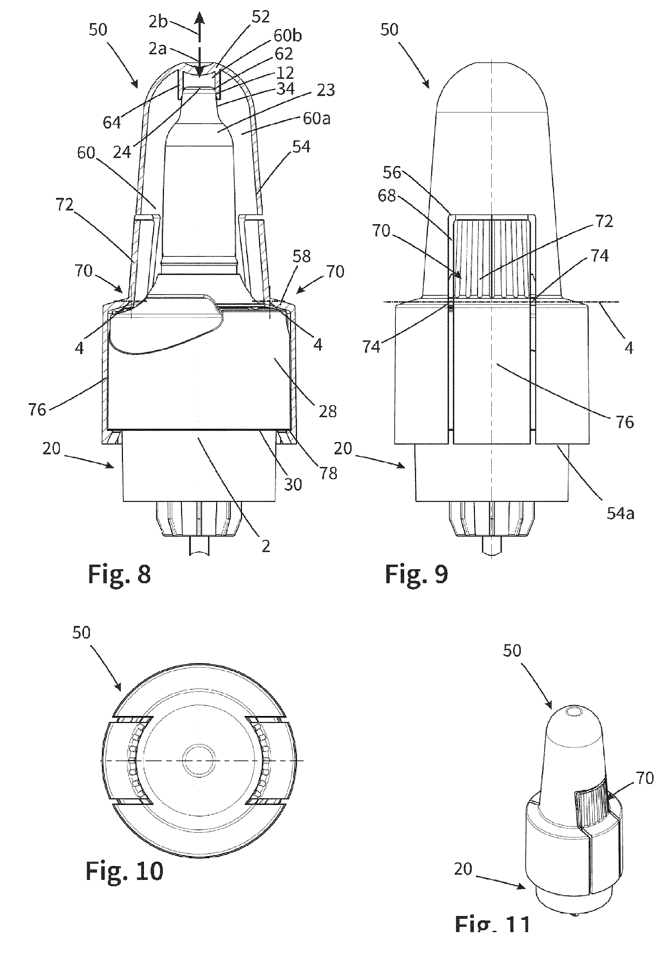

Diesbezüglich unterscheidet sich die Kappe des dritten Ausführungsbeispiels der Fig. 8 bis 11 von der vorangegangenen Kappe. Bei der Kappe 50 dieses Ausführungsbeispiels untergliedert sich der Kappeninnenraum 60 in einen belüfteten Bereich 60a, der durch die Freischnitte 68 hindurch mit einer äußeren Umgebung kommuniziert, sowie einen isolierten Bereich 60b, der bei aufgesetzter Kappe die Austragöffnung 24 umgibt. An der Innenseite der Stirnfläche 52 der Kappe 50 ist zu diesem Zweck ein ringförmiger Steg 64 vorgesehen. Im aufgesetzten Zustand der Kappe liegt eine Dichtfläche 62 dieses Ringsteges 64 an einer Dichtfläche 34 des Applikators 23 an. Die Dichtflächen und die Mittelachse 2 schließen einen Winkel von etwa 15° ein. Dies gestattet es bei ausreichend weichem Kunststoffwerkstoff insbesondere der Kappe 50, die Dichtigkeit im Kontaktbereich 12 in einem vergleichsweise großen Toleranzbereich bezogen auf die Einstecktiefe des als Nasenolive ausgebildeten Applikators 23 im Ringsteg 68 zu bewirken. Dies ist von Vorteil, um gleichzeitig auch ein einwandfreies und sicheres Einrasten der Halterasten 78 unter die Haltekante 30 zu bewirken, wenn die Kappe aufgesetzt wird.

Ein weiterer Unterschied zur vorausgegangenen Ausgestaltung der Kappe 50 liegt in deren grundsätzlicher Formgebung. Die Kappe 50 bei diesem Ausführungsbeispiel ist mit einer Stufe 58 versehen, an dem sich der Durchmesser der im Wesentlichen rotationssymmetrischen Kappe sprungartig aufweitet. Eine solche Gestaltung ist im Montagekontext von großem Vorteil, um die Kappen vereinzeln zu können. Die Torsionsbrücken 74, die die Kippachse 4 gemeinsam definieren, sind bei dieser Ausgestaltung im Bereich des geringeren Durchmessers angeordnet, so dass sie trotz der komplexeren Formgebung der Kappe 50 ein einwandfreies Verkippen der Wippelemente 70 gestatten.

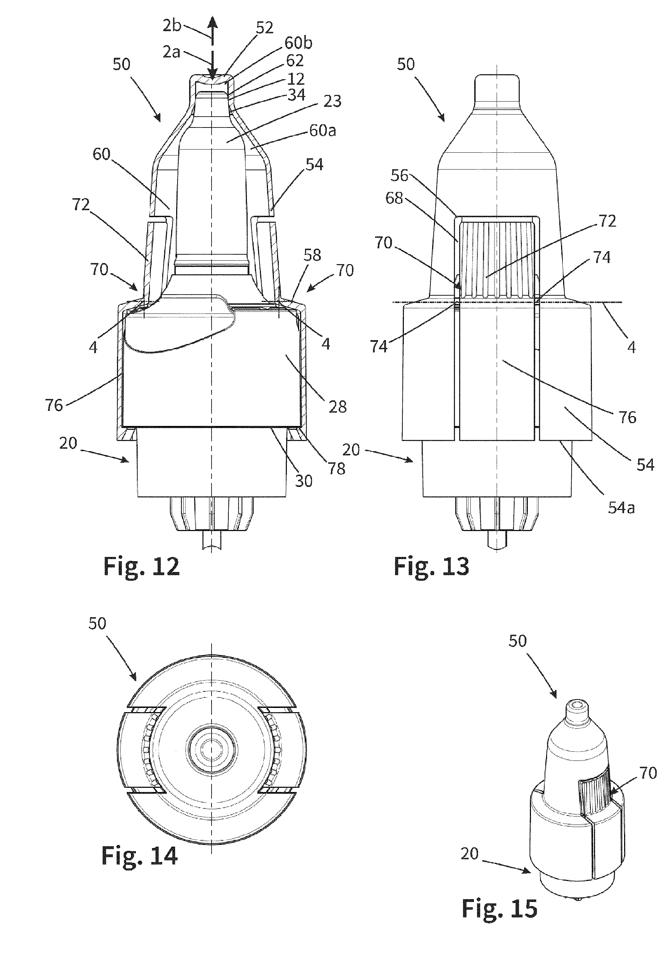

Beim Ausführungsbeispiel der Fig. 12 bis 15 weist die Kappe einen oberen Endbereich mit bereits von außen ersichtlicher deutlicher Verjüngung auf. Hierdurch ist es möglich, ohne einen produktionstechnisch nicht ganz einfachen Ringsteg 64 den genannten Kontaktbereich zu schaffen, wobei die vorteilhaften Winkel des vorangegangenen Ausführungsbeispiels erhalten bleiben. Die kappenseitige Dichtfläche 62 ist in diesem Fall unmittelbar an der Innenseite der Außenwandung der Kappe 50 vorgesehen.

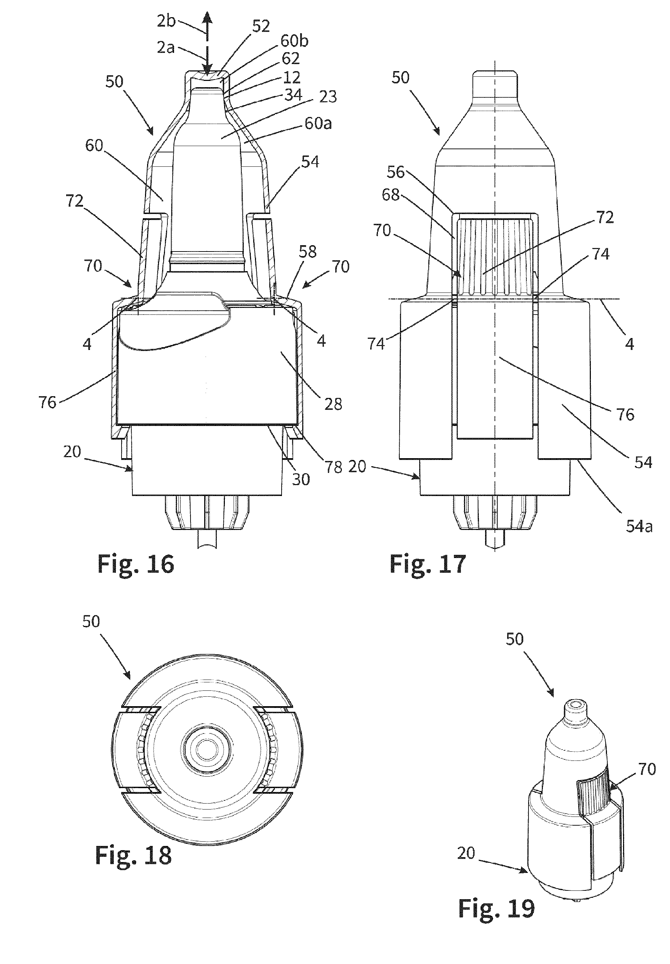

Bei der Ausgestaltung der Fig. 16 bis 19 ist gegenüber dem vorangegangenen Ausführungsbeispiel eine weitere Besonderheit vorgesehen. Wie insbesondere aus Fig. 17 ersichtlich ist, erstreckt sich die Mantelfläche 54 bei dieser Gestaltung weiter nach unten, so dass der untere Abschluss 54a der Mantelfläche 54 über den Rastschenkel 76 des Wippelements 70 hinausragt. Dies erschwert es für kleine Kinder, von unten unter das Wippelement 70 zu greifen und somit hier die Verrastung unmittelbar zu lösen.

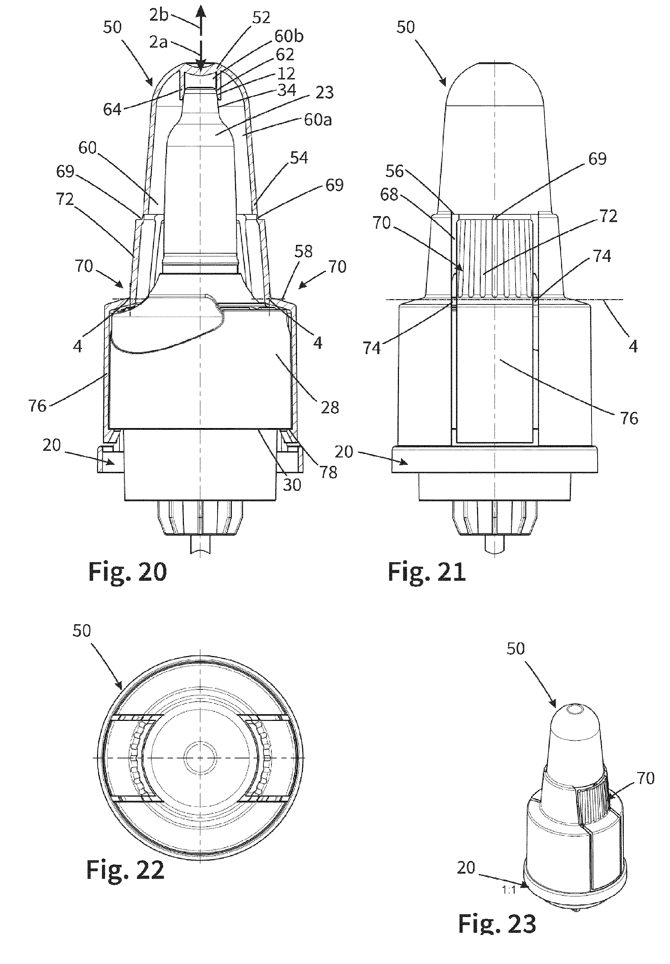

Bei der Ausgestaltung der Fig. 20 bis 23, die im Bereich der Austragöffnung über eine Kappenstruktur entsprechend dem Ausführungsbeispiel der Fig. 8 bis 11 verfügt, liegt eine Besonderheit darüber hinaus in der geschlossenen Ausgestaltung des Mantels 54, indem eine vollständig vom Mantel umschlossene Aussparung 56 vorgesehen ist, in der das Wippelement 70 angeordnet ist. Hierdurch wird es nochmals für kleine Kinder erschwert, ohne Begreifen des Mechanismus die Verrastung zu lösen.

Eine weitere Besonderheit dieser Ausgestaltung liegt im Vorhandensein der Sicherungsbrücken 69. Diese verbinden im Lieferzustand den Betätigungsschenkel 72 der Wippelemente 70 mit der Mantelfläche 54, so dass eine Kippbeweglichkeit der Wippelemente 70 im Lieferzustand noch nicht gegeben ist. Vielmehr muss zunächst die Brücke 69 durch kräftiges Eindrücken der Betätigungsschenkel 72 zerstört werden. Somit ist eine zusätzliche Kindersicherung für den Spender im Lieferzustand gegeben und zudem ein Originalitätsschutz, der unmittelbar erkennbar macht, ob die Kappe bereits abgenommen wurde.

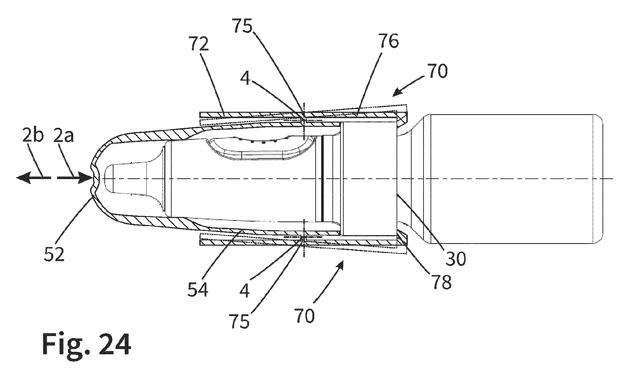

Alle bis hier beschriebenen Ausführungsbeispiele weisen eine Kappe auf, bei der die Wippelemente 70 bündig fluchtend in Aussparungen der Mantelfläche 54 eingesetzt sind, so dass der Kappeninnenraum zumindest teilweise belüftet ist. Auch wenn eine solche Gestaltung als produktionstechnisch vorteilhaft angesehen wird und insbesondere bei Ausgestaltungen, bei denen die Belüftung der Austragöffnung vorgesehen ist, als vorteilhaft angesehen wird, ist auch eine hiervon abweichende Gestaltung denkbar, wie in Fig. 24 gezeigt. Bei dieser Gestaltung ist die Mantelfläche 54 der Kappe umlaufend geschlossen. Die Wippelemente 70 sind bei dieser Gestaltung außenseitig an der Kappe 50 angebracht und durch elastische Kippstege 75 mit dieser verbunden. Diese Kippstege 75 definieren die Kippachsen 4, um die in der gestrichelt dargestellten Art und Weise die Wippelemente 70 verschwenkt werden können, um wiederum eine Verrastung der Halterasten 78 an der Haltekante 30 zu lösen.

## Patentansprüche

1. Spender (10) zum Austrag pharmazeutischer Flüssigkeiten mit den folgenden Merkmalen:
a. der Spender (10) umfasst eine Austragvorrichtung (20) mit einem Flüssigkeitsspeicher (22) und einer Austragsöffnung (24) zum Austrag der Flüssigkeit und
b. der Spender umfasst eine Kappe (50), die auf der Austragvorrichtung (20) in einer Aufsetzrichtung (2a) aufsetzbar ist und im aufgesetzten Zustand die Austragöffnung (24) überdeckt,
**gekennzeichnet durch** die zusätzlichen Merkmale:
c. die Kappe (50) verfügt über eine stirnseitige Deckfläche (52) und eine damit verbundene Mantelfläche (54) und
d. an der Mantelfläche (54) ist mindestens ein Wippelement (70) vorgesehen und
e. das Wippelement (70) ist stoffschlüssig und um eine Kippachse (4) schwenkbeweglich an der Mantelfläche (54) angebracht und
f. die Kippachse (4) unterteilt das Wippelement (70) in einen Betätigungsschenkel (72) und einen Rastschenkel (76), so dass das Niederdrücken des Betätigungsschenkels (72) eine gegengerichtete Verlagerung des Rastschenkels (76) bewirkt und
g. an der Innenseite des Rastschenkels (76) ist eine Halteraste (78) vorgesehen, die gemeinsam mit einer korrespondierenden Haltekante (30) an der Austragvorrichtung (20) formschlüssig ein Abziehen der Kappe (50) entgegen der Aufsetzrichtung (2a) verhindert.

2. Spender (10) nach Anspruch 1 mit dem folgenden zusätzlichen Merkmal:
a. die Kippachse (4) ist bezogen auf die Aufsetzrichtung (2a) tangential ausgebildet.

3. Spender (10) nach Anspruch 1 oder 2 mit den folgenden zusätzlichen Merkmalen:
b. in der Mantelfläche (54) ist eine Aussparung (56) vorgesehen und
c. in der Aussparung (56) ist das Wippelement (70) angeordnet und hierbei mittels eines Freischnittes (68) von der Mantelfläche (54) getrennt, wobei der Freischnitt (68) einen Kappeninnenraum (60) mit einer äußeren Umgebung kommunizierend verbindet, und
d. das Wippelement (70) ist im Bereich zweier Torsionsbrücken (74) mit der Mantelfläche (54) verbunden, wobei diese beiden Torsionsbrücken (74) die Kippachse (4) des Wippelements (70) definieren, um die das Wippelement (70) elastisch verkippbar ist.

4. Spender (10) nach Anspruch 3 mit dem zusätzlichen Merkmal:
a. das Wippelement (70) ist in der Aussparung (56) allseitig von der Mantelfläche (54) eingerahmt.

5. Spender (10) nach Anspruch 3 mit den folgenden zusätzlichen Merkmalen:
a. die Aussparung (56) erstreckt sich bis zu einem unteren Rand (54a) der Mantelfläche (54) und
b. das Wippelement (70) in der Aussparung (56) bildet im Bereich der Aussparung (56) das untere Ende der Kappe (50),
vorzugsweise zusätzlich mit dem Merkmal:
c. die Mantelfläche (54) beidseitig der Aussparung (56) erstreckt sich weiter nach unten als das Wippelement (70).

6. Spender (10) nach einem der vorstehenden Ansprüche mit dem zusätzlichen Merkmal:
a. das Wippelement (70) und die Mantelfläche (54) sind im Lieferzustand zusätzlich zur Verbindung im Bereich der Kippachse auch mittels einer Sicherungsbrücke (69) miteinander verbunden, die zum Zwecke des Austenkens des Wippelements (70) zum Abnehmen der Kappe (50) bestimmungsgemäß zerstört werden muss.

7. Spender (10) nach einem der vorstehenden Ansprüche mit den folgenden zusätzlichen Merkmalen:
a. die Kappe (50) weist eine Stufe (58) auf, die bezogen auf die Aufsetzrichtung (2a) durch einen Sprung im Querschnitt von einem kleineren Querschnitt zu einem größeren Querschnitt gebildet wird, und
b. die Kippachse (4) und insbesondere die zwei Torsionsbrücken (74) sind fluchtend mit dem kleineren Querschnitt angeordnet.

8. Spender (10) nach einem der vorstehenden Ansprüche mit dem zusätzlichen Merkmal:
a. die Innenseite der Kappe (50) und die Außenform der Austragvorrichtung (20) sind derart aufeinander angepasst, dass bei aufgesetzter Kappe ein Anliegen entlang eines umlaufend geschlossenen Kontaktbereichs (12) bewirkt wird, durch den die Austragöffnung (24) gegenüber einem mit der äußeren Umgebung kommunizierenden Bereich (60a) des Kappeninnenraums (60) isoliert ist.

9. Spender (10) nach einem der vorstehenden Ansprüche mit den folgenden zusätzlichen Merkmalen:
a. der umlaufende Kontaktbereich (12) wird auf Kappenseite durch einen kappenseitige erste Dichtfläche (34) und auf Seite der Austragvorrichtung durch eine austragsvorrichtungsseitige zweite Dichtflächen (62) gebildet, und
b. die Dichtflächen (34,62) schließen mit der Aufsetzrichtung (2a) einen Winkel von <20° ein.

10. Spender (10) nach einem der Ansprüche 8 und 9 mit dem zusätzlichen Merkmal:
a. die erste Dichtflächen (34) wird durch die Innenseite eines umlaufenden Ringsteges (64) gebildet, der im Bereich der stirnseitigen Deckfläche (52) innenseitig an der Kappe (50) angebracht ist.

11. Spender (10) nach einem der vorstehenden Ansprüche mit den folgenden zusätzlichen Merkmalen:
a. die Austragvorrichtung (20) umfasst eine Gehäuse (26),
b. die Austragvorrichtung umfasst eine Steuereinrichtung mit einer Pumpe oder einem Schaltventil, das durch eine erste Leitung mit dem Flüssigkeitsspeicher (22) und durch eine zweite Leitung mit der Austragöffnung (24) verbunden ist und mittels derer Flüssigkeit aus dem Flüssigkeitsspeicher (22) zur Austragöffnung (24) geleitet werden kann,
c. die Austragvorrichtung umfasst einen Betätigungsdrücker (28), der zum Zwecke des Flüssigkeitsaustrags kraftbeaufschlagbar ist und hierdurch die Steuereinrichtung betätigt.

12. Spender (10) nach Anspruch 11 mit dem zusätzlichen Merkmal:
a. die Austragöffnung (24) ist an einem Applikator (23) vorgesehen, der gemeinsam mit dem Betätigungsdrücker (28) verlagerbar ist, wobei am Applikator (23) auch die Haltekante (30) zur Festlegung der Kappe (50) vorgesehen ist.

13. Spender (10) nach Anspruch 11 mit den zusätzlichen Merkmalen:
a. die Austragöffnung (24) ist zum Austrag der Flüssigkeit in einer Austragrichtung (2b) ausgebildet,
b. der Betätigungsdrücker (28) ist seitlich in einer Aussparung (27) einer Mantelfläche des Gehäuses (26) vorgesehen und mit der Steuereinrichtung derart verbunden ist, dass ein bezogen auf die Austragrichtung (2b) radiales Eindrücken des Betätigungsdrückers (28) die Steuereinrichtung betätigt, so dass Flüssigkeit zurAustragöffnung (24) geleitet wird, und
c. die Kappe (50) überdeckt im aufgesetzten Zustand den Betätigungsdrücker (28).

14. Spender (10) nach einem der vorstehenden Ansprüche mit einem der folgenden Merkmale:
a. die Kappe weist ein Wippelement auf und die erforderliche Kraftbeaufschlagung des Betätigungsschenkels am kippachsenfernsten Punkt zur ausreichenden Auslenkung der Halteraste, um diese außer Eingriff von der Haltekante zu bringen, beträgt vorzugsweise mindestens 50 N, oder
b. die Kappe (50) weist zwei Wippelemente (70) auf und die erforderliche Kraftbeaufschlagung der jeweiligen Betätigungsschenkel (72) am jeweils kippachsenfernsten Punkt zur ausreichenden Auslenkung der Halterasten (78), um diese außer Eingriff von der Haltekante (30) zu bringen, beträgt vorzugsweise mindestens 30 N, oder
c. die Kappe weist mindestens drei Wippelemente auf.

15. Spender (10) nach einem der vorstehenden Ansprüche mit mindestens einem der zusätzlichen Merkmale:
a. die erforderliche Kraftbeaufschlagung des Betätigungsschenkels (72) am kippachsenfernsten Punkt zur ausreichenden Auslenkung der Halteraste (78), um diese außer Eingriff von der Haltekante (30) zu bringen, beträgt je Wippelement mindestens 50 N und/oder
b. der Betätigungsschenkels (72) ist mit einer Strukturierung, insbesondere mit einer Riffelung, versehen, und/oder
c. das Wippelement (70) ist fluchtend mit der Mantelfläche (54) in der Aussparung (56) der Mantelfläche (54) angeordnet und/oder
d. die Austragvorrichtung (20) ist zur nasalen Verabreichung der Flüssigkeit ausgebildet und weist hierfür einen als Nasenolive ausgebildeten Applikator (23) auf und/oder
e. die Kappe (50) weist eine im Wesentlichen rotationssymmetrische Mantelfläche (54) auf und/oder
f. die Haltekante (30) ist umlaufend an der Austragvorrichtung (20) vorgesehen, so dass die Kappe (50) in beliebiger Drehstellung aufsetzbar ist und/oder
g. der Flüssigkeitsspeicher (22) ist mit einer pharmazeutischen Flüssigkeit befüllt.
